(19) Europäisches Patentamt
European Patent Office
Office européen des brevets

(11) EP 1 205 179 A1

(12) **DEMANDE DE BREVET EUROPEEN**

(43) Date de publication:
**15.05.2002 Bulletin 2002/20**

(51) Int Cl.[7]: **A61K 7/48**

(21) Numéro de dépôt: **01402717.1**

(22) Date de dépôt: **19.10.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **10.11.2000 FR 0014479**

(71) Demandeur: **L'OREAL**
**75008 Paris (FR)**

(72) Inventeurs:
- **Chevalier, Véronique**
**94440 Villecresnes (FR)**
- **Pham, Dang-Man**
**94370 Sucy-en-Brie (FR)**

(74) Mandataire: **Renard, Emmanuelle**
**L'OREAL-DPI**
**6 rue Bertrand Sincholle**
**92585 Clichy Cedex (FR)**

(54) **Composition cosmétique comprenant un dérivé d'aminophénol et un isoflavonoide**

(57) L'invention concerne une composition comprenant, dans un milieu physiologiquement acceptable, au moins un dérivé d'aminophénol et au moins un isoflavonoïde.

L'invention concerne également l'utilisation de cette composition pour la fabrication d'une préparation dépigmentante et/ou blanchissante de la peau humaine.

Ces associations d'actifs présentent une synergie dans la dépigmentation ou le blanchiment de la peau.

EP 1 205 179 A1

## Description

La présente invention concerne une composition comprenant, dans un milieu physiologiquement acceptable, au moins un dérivé d'aminophénol et au moins un isoflavonoïde, ainsi que l'utilisation de cette composition dans une préparation cosmétique et/ou dermatologique dépigmentante et/ou blanchissante de la peau humaine.

Depuis plusieurs années, des efforts importants ont été déployés en vue de proposer des substances dépigmentantes topiques inoffensives présentant une bonne efficacité. L'utilisation de telles substances présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestroprogestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc) et pour les leucodermies, à défaut de pouvoir repigmenter la peau lésée, on achève de dépigmenter les zones de peau normale résiduelle pour donner à l'ensemble de la peau une teinte blanche homogène.

Divers agents dépigmentants ont ainsi été proposés dans l'art antérieur.
Parmi les plus connus, on peut citer : l'hydroquinone et ses dérivés tels que l'arbutine et ses esters ; l'acide ascorbique et ses dérivés, notamment glycosylés ; l'acide kojique et ses esters ; l'acide ellagique et ses dérivés ; les extraits de plantes, et notamment de réglisse, de mûrier ou de scutellaire ; ainsi que le glutathion, la cystéine et leurs précurseurs.

D'autre part, il a été démontré par la demanderesse que certains dérivés d'aminophénol avaient la propriété d'inhiber la mélanogénèse même à faibles concentrations, sans faire preuve de cytotoxicité (WO 99/10318).

La Demanderesse a maintenant découvert de manière inattendue que l'association d'un dérivé d'aminophénol avec un isoflavonoïde présentait un pouvoir dépigmentant supérieur à celui de ces composés, considérés séparément. En d'autres termes, cette association potentialise l'efficacité de ces composés qui produisent un effet de synergie dans le blanchiment et/ou la dépigmentation de la peau.

Les isoflavonoïdes constituent une sous-classe des flavonoïdes, formés d'un squelette 3-phénylchromane qui peut comporter des substituants variés et différents niveaux d'oxydation. Contrairement aux flavonoïdes, ils ne sont présents que dans un nombre très limité de plantes.

Le terme isoflavonoïde regroupe plusieurs classe de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les $\alpha$-méthyldeoxybenzoines ou encore les 2-arylbenzofuranes. A cet égard on se reportera avantageusement pour une revue complète sur les isoflavonoïdes, leurs méthodes d'analyse et leurs sources, au chapitre 5 "Isoflavonoïds" écrit par P.M. Dewick, dans <u>The Flavonoïds,</u> Harbone éditeur, pp. 125-157 (1988).

Certains isoflavonoïdes sont certes connus en tant que dépigmentants. Toutefois, à la connaissance de la Demanderesse, il n'a encore jamais été envisagé de les associer à des dérivés d'aminophénol.

La présente invention a donc pour objet une composition comprenant, dans un milieu physiologiquement acceptable, au moins un dérivé d'aminophénol et au moins un isoflavonoïde.

Par "milieu physiologiquement acceptable", on entend un milieu convenant à une application topique sur la peau ou ses phanères, c'est-à-dire compatible avec la peau et les ongles.

L'invention a également pour objet l'utilisation cosmétique de cette composition comme préparation dépigmentante et/ou blanchissante de la peau.

L'invention a en outre pour objet l'utilisation de cette composition pour la fabrication d'une préparation dépigmentante et/ou blanchissante de la peau humaine.

La présente invention se rapporte également à un procédé cosmétique de dépigmentation et/ou de blanchiment de la peau humaine consistant à appliquer sur la peau une composition selon l'invention.

Les composés aminophénol selon l'invention répondent préférentiellement à la formule (I) suivante :

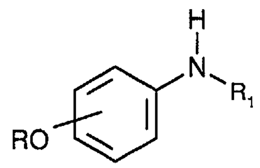

(I)

dans laquelle :

R représente un atome d'hydrogène ou un radical $-COR_2$,
avec $R_2$ représentant un radical choisi parmi un radical alkyle ou alkoxyle, saturé ou insaturé, linéaire, cyclique ou ramifié, en $C_1$ à $C_{30}$, éventuellement hydroxylé,

$R_1$ est choisi parmi un radical de formules (a), (b) ou (c) suivantes :

(a)$-CO-NR_3R_4$

(b) $-CO-O-R_5$

(c)$-SO_2-R_5$

avec $R_3$ représentant un atome d'hydrogène ou un radical alkyle en $C_1$ à $C_6$, linéaire ou ramifié, saturé ou insaturé, éventuellement hydroxylé,
avec $R_4$ représentant un atome d'hydrogène ou un radical choisi parmi un radical alkyle, saturé ou insaturé, linéaire, cyclique ou ramifié, en $C_1$ à $C_{30}$, éventuellement hydroxylé,
avec $R_5$ représentant un radical choisi parmi un radical alkyle, saturé ou insaturé, linéaire, cyclique ou ramifié, en $C_1$ à $C_{30}$, éventuellement hydroxylé,
ainsi que leurs sels d'addition avec un acide ou une base physiologiquement acceptable.

Ces composés présentent l'avantage d'être faciles à obtenir. Ils peuvent être notamment obtenus en faisant réagir un aminophénol avec une entité chimique activée, telle qu'un imidazolide ou un isocyanate, lorsque $R_1$ est un radical de formule (a), un chloroformiate, lorsque $R_1$ est un radical de formule (b), ou un chlorure de sulfonyle, lorsque $R_1$ est un radical de formule (c).

Parmi les radicaux alkyle linéaire ou ramifié ayant de 1 à 30 atomes de carbone, on peut citer avantageusement les radicaux méthyle, éthyle, propyle, isopropyle, butyle, tertiobutyle, hexyle, octyle, nonyle, 2-éthyl-hexyle, dodécyle, tridécyle, hexadécyle, béhényle, octadécyle, tétracosyle, hexacosyle, octacosyle, myricyle, 2-butyl-octyle, et 2-hexyl-décyle. De manière encore plus préférentielle, le radical alkyle comprend généralement de 1 à 6 atomes de carbone et peut être choisi parmi les radicaux méthyle, éthyle, propyle, isopropyle, tertiobutyle, et hexyle.

Lorsque le radical alkyle est insaturé, on préfère un radical présentant une ou plusieurs insaturations éthyléniques, tel que les radicaux neryl, 2-nonyl 2-butényl, 6-(1,3-pentadiényl)-2,4,7-dodécanetrièn-9-ynyl et plus particulièrement le radical allyle.

Lorsque le radical alkyle est cyclique, on peut notamment citer le radical cyclohexyle, cholestéryle ou terbutylcyclohexyle.

Lorsqu'il est hydroxylé, le radical comprend de préférence 1 à 6 atomes de carbone et 1 à 5 groupes hydroxyles.

Parmi les radicaux monohydroxyalkyle, on préfère un radical contenant de préférence 1 à 3 atomes de carbone, notamment les radicaux hydroxyméthyl, 2-hydroxyéthyl, 2- ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle et 2,3,4,5,6-pentahydroxyhexyle.

Les radicaux alkoxylés sont des radicaux alkyles, tels que notamment décrits ci-dessus, précédés d'un atome

d'oxygène.

Avantageusement, on choisira les dérivés d'aminophénol de formule (I) de telle sorte que l'une au moins et de préférence toutes les conditions ci-dessous soient respectées :

- R représente un atome d'hydrogène,
- la fonction -OR sur le radical phényl est en position ortho ou avantageusement en position para,
- $R_1$ est choisi parmi un radical de formules (a) ou (b).

Les composés de formule (I) selon l'invention sont plus particulièrement choisis parmi le N-éthyloxycarbonyl-4-aminophénol ; le N-éthyloxycarbonyl-O-éthyloxycarbonyl-4-aminophénol ; le N-cholestéryloxycarbonyl-4-aminophénol et le N-éthylaminocarbonyl-4-aminophénol.

Dans le cadre de la présente invention, on utilise de préférence le N-éthyloxycarbonyl-4-aminophénol.

Les isoflavonoïdes convenant à une mise en oeuvre dans la présente invention peuvent être d'origine naturelle ou synthétique. Par "origine naturelle", on entend l'isoflavonoïde à l'état pur ou en solution à différentes concentrations, obtenu par différents procédés d'extraction à partir d'un élément, généralement une plante, d'origine naturelle. Par "origine synthétique", on entend l'isoflavonoïde à l'état pur ou en solution à différentes concentrations, obtenu par synthèse chimique.

On préfère utiliser les isoflavonoïdes d'origine naturelle. Parmi ceux-ci, on peut citer : la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites, et leurs mélanges.

Les isoflavones sont préférées pour une utilisation dans la présente invention. Par ce terme, on entend aussi bien les formes aglycones (daidzéine, génistéine, glycitéine) que les formes glycosylées (daidzine, génistine, glycitine) des isoflavones.

Des procédés de préparation d'isoflavones sont notamment décrits dans WO 95/10530, WO 95/10512, US-5,679,806, US-5,554,519, EP-812 837 et WO 97/26269.

De façon particulièrement avantageuse on préfèrera utiliser un extrait de soja titré à 37% en isoflavones, commercialisé par la société MARUZEN sous la dénomination FUJIFLAVONES P40.

Le dérivé d'aminophénol et l'isoflavonoïde sont présents, dans la composition selon l'invention, en une quantité telle qu'ils agissent en synergie pour conférer à la composition un effet dépigmentant supérieur à celui obtenu avec une composition ne renfermant que l'un de ces composés.

Ainsi les dérivés d'aminophénol peuvent être notamment présents dans la composition en une quantité allant de 0,01 à 10 % en poids, et de préférence de 0,5 à 3 % du poids total de la composition.

De son côté l'isoflavonoïde représente de préférence de 0,0001% à 10%, et de préférence de 0,001 à 1%, du poids total de la composition. Bien entendu, si l'isoflavonoïde est présent sous forme de solution contenant un extrait de plante, l'homme du métier saura ajuster la quantité de cette solution dans la composition selon l'invention, de façon à obtenir les gammes de concentrations ci-dessus en isoflavonoïde.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage pour la peau.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association d'agents dépigmentants selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline) ainsi que leurs esters, les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, la composition comprend de préférence au moins un filtre UV et/ou un agent kératolytique et/ou desquamant.

Comme filtre UV utilisable selon l'invention, on peut citer par exemple et de façon non limitative les familles suivantes (les noms correspondent à la nomenclature CTFA des filtres) :

les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène-dicampho-sulfonique , et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinnoxate, le DEA méthoxycinnamate, le méthylcinnamate de diisopropyle, le glucéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'éthylhexylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salycilates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba).

L'invention va maintenant être illustrée à l'aide des exemples non limitatifs qui suivent.

**Exemple 1 : Mise en évidence de l'effet synergique des composés selon l'invention**

Un test biologique in vitro (inhibition de l'activité de la tyrosinase de champignon) a mis en évidence l'activité dépigmentante des associations de composés selon l'invention.

Ce test consiste à mesurer l'absorbance (densité optique DO) à 475 nm en fonction du temps pour chacun des actifs dépigmentants testés.

Ceux-ci sont dissous (à différentes concentrations) dans un solvant approprié en présence d'un tampon phosphate, de 1 ml de L-tyrosine à 2,0 x $10^{-3}$ M dans un tampon phosphate, de 0,1 ml de L-Dopa à 1,0 x $10^{-4}$ M dans un tampon phosphate et de 50 µl de solution de tyrosinase de champignon à 1 g/l dans un tampon phosphate, comparés à un témoin constitué du solvant utilisé à la même concentration dans le tampon phosphate. Un pourcentage d'inhibition est ensuite déterminé par l'équation suivante :

$$\%\text{inhibition} = \frac{DO_{max,\ 475\ nm}\ (\text{témoin}) - DO_{max,\ 475\ nm}\ (\text{actif})}{DO_{max,\ 475\ nm}\ (\text{témoin})} \times 100$$

Pour chacun des composés actifs étudiés, on détermine ensuite la concentration molaire pour laquelle devrait être observée une valeur d'inhibition proche de 25% (IC théorique). On obtient ainsi les concentrations $C_1$ et $C_2$ correspondant à chaque actif utilisé seul. On vérifie alors la valeur d'inhibition effectivement obtenue aux concentrations $C_1$ et $C_2$ (IC obtenu).
On réalise ensuite le mélange de ces deux actifs dans les concentrations $C_1$ et $C_2$ précédemment trouvées. On calcule alors le pourcentage d'inhibition théorique du mélange (ou IC théorique), correspondant à la somme des pourcentages d'inhibition observés pour les composés individuels, et on mesure le pourcentage d'inhibition obtenu (ou IC mesuré) dans les mêmes conditions expérimentales que pour les composés testés séparément.
A titre d'exemple les résultats obtenus avec le N-éthyloxycarbonyl-4-aminophénol et un extrait de soja titré à 37% en isoflavones (commercialisé par la société MARUZEN sous la dénomination FUJIFLAVONE P40) sont les suivants :

| Actifs | Concentration C | IC théorique (%) | IC obtenu (%) |
|---|---|---|---|
| N-Ethoxycarbonyl-4-aminophénol / éthanol | $C_1$=1,25 x $10^{-4}$ M | 24,8 | 23,5 |
| Extrait de soja (Fujiflavone P40) / DMSO | $C_2$=0,04 % | 24,0 | 31,4 |

| Association des actifs | IC théorique attendu (%) | IC mesuré (%) |
|---|---|---|
| N-Ethoxycarbonyl-4-aminophénol <u>et</u> Extrait de soja | 54,9 | 72,6 |

Il apparaît clairement que l'IC mesuré est nettement supérieur à l'IC théorique pour le mélange d'actifs.
Compte tenu de la précision de ce test (de l'ordre de 5%), l'effet de synergie des deux composés en association est ainsi clairement mis en évidence.

**Exemple 2 : Fluide H/E type oléosomes**

On prépare la composition suivante.
Les quantités sont indiquées en pourcentage pondéral.

| *Phase A* | |
|---|---|
| N-éthyloxycarbonyl-4-aminophénol | 0,5 % |
| Heptanoate de stéaryle et octanoate de stéaryle | 5,5 % |
| Huiles végétales | 11,6 % |
| Filtre UVA | 1,9 % |
| Cyclopentasiloxane | 3,7 % |
| Tristéarate de sucrose | 2 % |
| Monostéarate de sorbitane oxyéthyléné (4 OE) | 1,35 % |
| Acide stéarique | 1 % |
| Huile de ricin hydrogénée oxyéthylénée (60 OE) | 2,5 % |
| Acétate de tocophéryle | 0,5 % |

| *Phase B* | |
|---|---|
| FUJIFLAVONE P40 (MARUZEN) | 0,1 % |
| Conservateurs | 0,29 % |
| Triéthanolamine | 0,50 % |
| EDTA disodique | 0,05 % |
| Glycérine | 3 % |
| Eau qsp | 100 % |

On obtient un fluide blanchissant pour le visage.
Il peut être obtenu de la manière suivante : les phases A et B sont chauffées séparément à 65°C, puis refroidies à température ambiante. La phase B est alors introduite dans la phase A pour obtenir une émulsion E/H qui est ensuite

passée à l'homogénéisateur haute pression (500b, 1 à 3 passages).

**Exemple 3 : Fluide H/E type oléosomes**

| *Phase A* | |
|---|---|
| N-éthyloxycarbonyl-4-aminophénol | 0,5 % |
| Heptanoate de stéaryle et octanoate de stéaryle | 5,5 % |
| Huiles végétales | 11,6 % |
| Filtre UVA | 1,9 % |
| Cyclopentasiloxane | 3,7 % |
| Tristéarate de sucrose | 2 % |
| Monostéarate de sorbitane oxyéthyléné (4 OE) | 1,35 % |
| Acide stéarique | 1 % |
| Huile de ricin hydrogénée oxyéthylénée (60 OE) | 2,5 % |
| Acétate de tocophéryle | 0,5 % |

| *Phase B* | |
|---|---|
| FLAVOSTERONE SB (Ichimaru Pharcos) | 10,0 % |
| Conservateurs | 0,29 % |
| Triéthanolamine | 0,50 % |
| EDTA disodique | 0,05 % |
| Glycérine | 3 % |
| Eau qsp | 100 % |

Ce fluide blanchissant pour le visage peut être obtenu selon le même procédé que dans l'exemple 2.

**Exemple 4 : gel dépigmentant**

| *Phase A* | |
|---|---|
| Filtre UVB | 0,5 % |
| Cyclohexasiloxane | 5 % |

| *Phase B* | |
|---|---|
| SOY LIFE 150 NGMO (Schouton Products) | 0,5 % |
| Conservateurs | 0,65 % |
| EDTA disodique | 0,1 % |
| Hydroxyde de sodium | 0,11 % |
| Glycérine | 10 % |
| Ethanol | 2,5 % |
| N-éthyloxycarbonyl-4-aminophénol | 0,5 % |
| Eau qsp | 100 % |

| *Phase C* | |
|---|---|
| Carbomers | 0,4 % |

(suite)

| *Phase C* | |
|---|---|
| Eau | 14,6 % |

Ce gel peut être obtenu de manière classique pour l'homme du métier.

**Exemple 5 : émulsion H/E sans tensioactif**

| *Phase A* | |
|---|---|
| N-éthyloxycarbonyl-4-aminophénol | 0,5 % |
| Huiles végétales | 12 % |
| Filtre UVA | 2 % |
| Filtres UVB | 4 % |
| Cyclopentasiloxane | 6 % |

| *Phase B* | |
|---|---|
| FLAVOSTERONE SB (Ichimaru Pharcos) | 2 % |
| EDTA disodique Copolymère diglycol/CHDM/isophtalates/SIP | 0,05 % |
| (Eastman AQ 38S d'EASTMAN CHEMICAL) | 2 % |
| Glycérine | 5 % |
| Ethanol | 10 % |
| Eau qsp | 100 % |

On obtient une crème blanchissante convenant particulièrement bien aux peaux sensibles ou réactives.
Cette crème peut être obtenue de manière classique pour l'homme du métier.

## Revendications

1. Composition comprenant, dans un milieu physiologiquement acceptable, au moins un dérivé d'aminophénol et au moins un isoflavonoïde.

2. Composition selon la revendication 1, **caractérisée en ce que** le dérivé d'aminophénol est représenté par la formule (I) suivante :

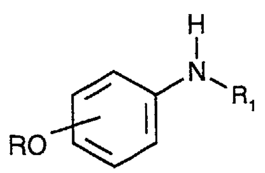

(I)

dans laquelle :

R représente un atome d'hydrogène ou un radical $-COR_2$,
avec $R_2$ représentant un radical choisi parmi un radical alkyle ou alkoxyle, saturé ou insaturé, linéaire, cyclique ou ramifié, en $C_1$ à $C_{30}$, éventuellement hydroxylé,

$R_1$ est choisi parmi un radical de formules (a), (b) ou (c) suivantes :

$$(a)\text{-}CO\text{-}NR_3R_4$$

$$(b)\ \text{-}CO\text{-}O\text{-}R_5$$

$$(c)\text{-}SO_2\text{-}R_5$$

avec $R_3$ représentant un atome d'hydrogène ou un radical alkyle en $C_1$ à $C_6$, linéaire ou ramifié, saturé ou insaturé, éventuellement hydroxylé,

avec $R_4$ représentant un atome d'hydrogène ou un radical choisi parmi un radical alkyle, saturé ou insaturé, linéaire, cyclique ou ramifié, en $C_1$ à $C_{30}$, éventuellement hydroxylé,

avec $R_5$ représentant un radical choisi parmi un radical alkyle, saturé ou insaturé, linéaire, cyclique ou ramifié, en $C_1$ à $C_{30}$, éventuellement hydroxylé.

**3.** Composition selon la revendication 1 ou 2, **caractérisée en ce que** ledit dérivé d'aminophénol présente l'une au moins et de préférence toutes les conditions ci-dessous :

- R représente un atome d'hydrogène,
- la fonction -OR sur le radical phényl est en position ortho ou avantageusement en position para,
- $R_1$ est choisi parmi un radical de formules (a) ou (b).

**4.** Composition selon l'une quelconque des revendications 1 à 3, **caractérisée en ce que** ledit dérivé d'aminophénol est choisi parmi le N-éthyloxycarbonyl-4-aminophénol; le N-éthyloxycarbonyl-O-éthyloxycarbonyl-4-aminophénol; N-cholestéryloxycarbonyl-4-aminophénol et le N-éthylaminocarbonyl-4-aminophénol.

**5.** Composition selon l'une quelconque des revendications 1 à 4, **caractérisée en ce que** ledit dérivé d'aminophénol est le N-éthyloxycarbonyl-4-aminophénol.

**6.** Composition selon l'une quelconque des revendications 1 à 5, **caractérisée en ce qu'**elle renferme de 0,01% à 10% en poids de dérivé d'aminophénol par rapport au poids total de la composition.

**7.** Composition selon la revendication 6, **caractérisée en ce qu'**elle renferme de 0,5% à 3% en poids de dérivé d'aminophénol par rapport au poids total de la composition.

**8.** Composition selon l'une quelconque des revendications 1 à 7, **caractérisée en ce qu'**elle renferme de 0,0001% à 10% en poids d'isoflavonoïde par rapport au poids total de la composition.

**9.** Composition selon la revendication 8, **caractérisée en ce qu'**elle renferme de 0,001% à 1% en poids d'isoflavonoïde par rapport au poids total de la composition.

**10.** Composition selon l'une quelconque des revendications précédentes, **caractérisée en ce que** ledit isoflavonoïde est choisi parmi les isoflavonoïdes d'origine naturelle.

**11.** Composition selon l'une quelconque des revendications précédentes, **caractérisée en ce que** ledit isoflavonoïde est choisi parmi la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites, et leurs mélanges.

**12.** Composition selon l'une quelconque des revendications précédentes, **caractérisée en ce que** ledit isoflavonoïde est choisi parmi les isoflavones.

**13.** Composition selon l'une des revendications 1 à 12, **caractérisée en ce qu'**elle renferme en outre au moins un filtre UV et/ou au moins un agent kératolytique et/ou desquamant.

**14.** Utilisation cosmétique de la composition selon l'une quelconque des revendications 1 à 13 comme préparation dépigmentante et/ou blanchissante de la peau.

**15.** Utilisation de la composition selon l'une quelconque des revendications 1 à 13 pour la fabrication d'une préparation dépigmentante et/ou blanchissante de la peau humaine.

**16.** Procédé cosmétique de dépigmentation et/ou de blanchiment de la peau humaine, consistant à appliquer la composition selon l'une quelconque des revendications 1 à 13 sur la peau.

Office européen des brevets

# RAPPORT DE RECHERCHE EUROPEENNE

Numéro de la demande
EP 01 40 2717

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | US 5 858 997 A (CROTTY BRIAN ANDREW ET AL) 12 janvier 1999 (1999-01-12)<br>* revendication 1 *<br>--- | 1,14 | A61K7/48 |
| Y | PATENT ABSTRACTS OF JAPAN<br>vol. 008, no. 071 (C-217),<br>3 avril 1984 (1984-04-03)<br>& JP 58 225004 A (ICHIMARU FUARUKOSU KK),<br>27 décembre 1983 (1983-12-27)<br>* abrégé *<br>--- | 1,14 | |
| A | PATENT ABSTRACTS OF JAPAN<br>vol. 1995, no. 02,<br>31 mars 1995 (1995-03-31)<br>& JP 06 321752 A (KAO CORP),<br>22 novembre 1994 (1994-11-22)<br>* abrégé *<br>--- | 1,14 | |
| A | PATENT ABSTRACTS OF JAPAN<br>vol. 1995, no. 09,<br>31 octobre 1995 (1995-10-31)<br>& JP 07 157494 A (KAO CORP),<br>20 juin 1995 (1995-06-20)<br>* abrégé *<br>--- | 1,14 | **DOMAINES TECHNIQUES RECHERCHES (Int.Cl.7)**<br>A61K |
| A | PATENT ABSTRACTS OF JAPAN<br>vol. 2000, no. 01,<br>31 janvier 2000 (2000-01-31)<br>& JP 11 269066 A (KAO CORP),<br>5 octobre 1999 (1999-10-05)<br>* abrégé *<br>--- | 1,14 | |
| A | DE 42 27 806 A (FISCHER PHARMA LTD)<br>25 février 1993 (1993-02-25)<br>* revendications 1,4,10,11 *<br>---<br>-/-- | 1,14 | |

Le présent rapport a été établi pour toutes les revendications

| Lieu de la recherche | Date d'achèvement de la recherche | Examinateur |
|---|---|---|
| LA HAYE | 6 mars 2002 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 01 40 2717

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | PATENT ABSTRACTS OF JAPAN<br>vol. 1997, no. 07,<br>31 juillet 1997 (1997-07-31)<br>& JP 09 077655 A (MIKIMOTO PHARMACEUT CO LTD), 25 mars 1997 (1997-03-25)<br>* abrégé *<br>--- | 1,14 | |
| D,A | WO 99 10318 A (OREAL ;TULOUP REMY (FR); PHILIPPE MICHEL (FR); BLAISE CHRISTIAN (F)<br>4 mars 1999 (1999-03-04)<br>* revendications 1,9 *<br>--- | 1,14 | |
| P,A | EP 1 110 537 A (OREAL)<br>27 juin 2001 (2001-06-27)<br>* revendications 1,11 *<br>--- | 1,14 | |
| P,A | FR 2 796 838 A (OREAL)<br>2 février 2001 (2001-02-02)<br>* revendications 1,31 *<br>----- | 1,14 | |
| | | | DOMAINES TECHNIQUES RECHERCHES (Int.Cl.7) |

Le présent rapport a été établi pour toutes les revendications

| Lieu de la recherche | Date d'achèvement de la recherche | Examinateur |
|---|---|---|
| LA HAYE | 6 mars 2002 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 01 40 2717

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

06-03-2002

| Document brevet cité au rapport de recherche | Date de publication | Membre(s) de la famille de brevet(s) | Date de publication |
|---|---|---|---|
| US 5858997 A | 12-01-1999 | AUCUN | |
| JP 58225004 A | 27-12-1983 | JP 1294411 C | 26-12-1985 |
| | | JP 60019885 B | 18-05-1985 |
| JP 06321752 A | 22-11-1994 | AUCUN | |
| JP 07157494 A | 20-06-1995 | AUCUN | |
| JP 11269066 A | 05-10-1999 | AUCUN | |
| DE 4227806 A | 25-02-1993 | IL 99291 A | 15-04-1997 |
| | | AU 654030 B2 | 20-10-1994 |
| | | AU 2122092 A | 25-02-1993 |
| | | CA 2076467 A1 | 24-02-1993 |
| | | CH 684739 A5 | 15-12-1994 |
| | | DE 4227806 A1 | 25-02-1993 |
| | | ES 2050074 A1 | 01-05-1994 |
| | | FR 2680466 A1 | 26-02-1993 |
| | | GB 2259014 A ,B | 03-03-1993 |
| | | PT 100800 A ,B | 28-02-1994 |
| JP 09077655 A | 25-03-1997 | AUCUN | |
| WO 9910318 A | 04-03-1999 | FR 2767823 A1 | 05-03-1999 |
| | | BR 9806236 A | 21-03-2000 |
| | | CN 1079393 T | 20-02-2002 |
| | | CN 1237155 T | 01-12-1999 |
| | | EP 0966434 A1 | 29-12-1999 |
| | | WO 9910318 A1 | 04-03-1999 |
| | | JP 2000508351 T | 04-07-2000 |
| EP 1110537 A | 27-06-2001 | FR 2802415 A1 | 22-06-2001 |
| | | CN 1302599 A | 11-07-2001 |
| | | EP 1110537 A2 | 27-06-2001 |
| | | JP 2001172173 A | 26-06-2001 |
| | | US 2001053350 A1 | 20-12-2001 |
| FR 2796838 A | 02-02-2001 | FR 2796838 A1 | 02-02-2001 |

EPO FORM P0460

Pour tout renseignement concernant cette annexe : voir Journal Officiel de l'Office européen des brevets, No.12/82